# EUROPÄISCHE PATENTSCHRIFT

(11) **EP 2 462 218 B1**
(45) Veröffentlichungstag und Bekanntmachung des Hinweises auf die Patenterteilung: **25.12.2013**
(21) Anmeldenummer: 10739641.8
(22) Anmeldetag: 06.08.2010
(51) Int. Cl.: C12M 1/00

(54) **Bioreaktor aus Silikonmaterialien**
Bioreactor consisting of silicon materials
Boiréacteur en matériaux siliconés

(30) Priorität: 07.08.2009 DE 102009028339
(43) Veröffentlichungstag der Anmeldung: 13.06.2012
(73) Patentinhaber: Wacker Chemie AG, 81737 München (DE)
(72) Erfinder: MÜLLER-REES, Christoph, 82049 Pullach (DE); PFALLER, Rupert, 80639 München (DE)
(74) Vertreter: Schuderer, Michael
(86) Internationale Anmeldenummer: PCT/EP2010/061487
(87) Internationale Veröffentlichungsnummer: WO 2011/015653

(56) Entgegenhaltungen:
- WO-A1-2007/129327
- WO-A1-2008/145719
- DE-A1- 19 850 607
- US-A1- 2009 011 492

## Beschreibung

Die Erfindung betrifft einen Bioreaktor, welcher mit Siliconen ausgerüstet ist, Verfahren zur Herstellung von Bioreaktoren, sowie die Verwendung von Siliconen zur Herstellung von Bioreaktoren.

Bioreaktoren werden zur großtechnischen Produktion von phototrophen Organismen, z.B. Cyanobakterien oder Mikroalgen, beispielsweise Spirulina, Chlorella, Clamydomonas oder Haematococcus eingesetzt. Derartige Mikroalgen sind in der Lage, mit Hilfe von Lichtenergie, CO₂ und Wasser in Biomasse umzuwandeln. Photobioreaktoren der ersten Generation nutzen das Sonnenlicht als Lichtquelle. Die Reaktoren bestehen aus großen offenen Beckenanlagen in vielerlei Formgestalt, beispielsweise Rundbeckenanlagen mit Durchmessern bis zu 45 m und umlaufenden Mischarmen. Diese Reaktoren sind im Allgemeinen aus Beton oder Kunststoffen gefertigt. Geschlossene Bioreaktoren werden ebenfalls in vielerlei Formen eingesetzt. Bei den geschlossenen Bioreaktoren kann es sich um Plattenbioreaktoren, Rohrbioreaktoren, (Blasen)Säulenbioreaktoren oder Schlauchbioreaktoren handeln. Dieser Reaktortyp wird aus transparenten oder transluzenten Materialien gefertigt, wie Glas oder Kunststoff.

Die Kultivierungsbedingungen phototropher Mikroorganismen, die in geschlossenen Reaktoren hergestellt werden, sind bis dato über einen längeren Zeitraum nicht konstant zu halten, da die in einer Kultivierungsphase sich bildenden phototrophen Mikroorganismen an den Reaktorwänden ablagern, was zu Schwankungen der eingetragenen Lichtmenge in das Kulturmedium sowie zu einer unsteten Durchmischung des Kulturmediums führt. Algenanlagerungen werden häufig durch Stressbedingungen während der Kultivierung hervorgerufen, deren Ursachen unkontrollierte Wachstumsbedingungen (z.B. Licht, Temperatur bei Open-Pondsowie bei geschlossenen Reaktoren) der Mikroorganismen oder die Induktion der Produktion von Wertstoffen durch die phototrophen Organismen (z.B. Astaxanthin, beta-Carotin) sein können.

Die WO 2007/129327 A1 betrifft einen Photobioreaktor zur Kultivierung von Biomasse, welcher aus transparenten, helikal gewickelten Rohren aufgebaut ist. Als Rohrmaterial wird allgemein Silicon empfohlen, wobei auf die Anwuchsproblematik nicht eingegangen wird. In der WO 2008/145719 wird die Ausleuchtung von Photoreaktoren mit LED-Kunststoff-Formteilen beschrieben. Als Reaktormaterialien werden Stahl, Kunststoff und Keramik aufgezählt. Das Beleuchtungselement ist vorzugsweise ein LED-Silicon-Formteil. Die WO 2004/108881 A2 beinhaltet eine Bioreaktor-Anordnung aus Behälter und Lichtquelle, wobei als Materialien für den Behälter alle möglichen Kunststoffe, unter anderem nicht näher spezifizierte Silicone angegeben werden. Die WO 2009/037683 A1 beschreibt einen wannenförmigen Bioreaktor mit schirmförmiger Abdeckung aus lichtdurchlässigen, nicht näher spezifizierten Materialien. Zur Zufuhr von Kohlendioxid werden gasdurchlässige Schläuche, vorzugsweise aus Silicon eingesetzt. Die GB 2118572 A beschreibt einen Photobioreaktor mit Glasröhren, welche mit U-förmigen Verbindungsstücken aus Silicon verbunden sind. In der DE 10 2005 025 118 A1 wird ein aus Glasrohren gefertigter Photobioreaktor beschrieben, wobei Mikroorganismen, welche sich an Oberflächen abgelagert haben, mittels Ultraschall entfernt werden. In der US 2003/0073231 A1 wird ein aus Thermoplasten wie Polyvinylchlorid oder Polyethylen gefertigter Photobioreaktor beschrieben. Gegenstand der US 2007/0048848 A1 ist ein ebenfalls aus Thermoplasten gefertigter Photobioreaktor. In beiden Fällen werden Anlagerungen von Mikroorganismen an den Reaktorwänden mittels mechanischer Mittel beseitigt, beispielsweise Bürsten. Es handelt sich hierbei in allen Fällen um relativ aufwändige Verfahren, welche nicht beliebig skalierbar sind. In der DE 44 16 069 A1 wird empfohlen, lichtleitende Fasern, welche zur Ausleuchtung von Bioreaktoren eingesetzt werden, mit einer glatten Oberfläche zu versehen. Die US 2008/0311649 A1 schlägt vor, in Röhren-Bioreaktoren die Durchflußgeschwindigkeit des Algen enthaltenden Mediums zu erhöhen, um die Algenablagerung zu verhindern. Nachteilig ist hierbei, dass die Kultivierungsparameter bezüglich der Strömungsgeschwindigkeit nicht mehr unabhängig einstellbar sind.

Vor diesem Hintergrund bestand die Aufgabe, Bioreaktoren zur Kultivierung von Mikroorganismen dahingehend zu verbessern, dass der Bewuchs mit Mikroorganismen an den mit dem Kultivierungsmedium in Kontakt tretenden Reaktorteilen weitgehend verhindert wird, und eventuell auftretender Bewuchs ohne hohen Aufwand entfernt werden kann. Die Lösung sollte die Produktqualität nicht negativ beeinflußen, upskalierbar sein, und unabhängig von den für die Kultivierung erforderlichen Prozess-Parametern universell einsetzbar sein.

Gegenstand der Erfindung ist ein Bioreaktor zur Kultivierung von phototrophen Organismen in einem wässrigen Kultivierungsmedium, dessen mit dem Kultivierungsmedium in Kontakt kommende Reaktorwandungen, ganz oder teilweise, aus Siliconmaterialien gefertigt sind, dadurch gekennzeichnet, dass die Siliconmaterialien aus additionsvernetzten Siliconen gefertigt sind, und die Oberfläche der Siliconmaterialien einen Kontaktwinkel zu Wasser von mindestens 100° aufweist.

Zur Kultivierung geeignete Organismen sind insbesondere phototrophe Makro- oder Mikroorganismen. Als phototrophe Organismen werden dabei solche bezeichnet, welche Licht und Kohlendioxid, oder gegebenenfalls noch eine weitere Kohlenstoffquelle, zum Wachstum benötigen. Beispiele für phototrophe Makroorganismen sind Makroalgen, Pflanzen, Moose, Pflanzenzellkulturen. Beispiele für phototrophe Mikroorganismen sind phototrophe Bakterien wie Purpurbakterien, phototrophe Mikroalgen einschließlich Cyanobakterien. Bevorzugt dient der Bioreaktor zur Kultivierung von phototrophen Mikroorganismen, besonders bevorzugt zur Kultivierung phototropher Mikroalgen.

Bei dem Bioreaktor kann es sich um einen geschlossenen Reaktor oder einen offenen Reaktor, jeweils mit beliebiger Formgestalt, handeln. Bei den offenen Reaktoren können beispielsweise Wannen oder sogenannte "open ponds" oder "raceway ponds" eingesetzt werden. Als Bioreaktoren werden geschlossene Reaktoren bevorzugt. Bei den geschlossenen Bioreaktoren kann es sich beispielsweise um Plattenbioreaktoren, Rohrbioreaktoren, (Blasen)Säulenbioreaktoren oder Schlauchbioreaktoren handeln. Plattenbioreaktoren bestehen aus senkrecht oder geneigt angeordneten quaderförmigen Platten, wobei eine Vielzahl von Platten zu einem größeren Reaktorsystem verbunden ist. Rohrbioreaktoren bestehen aus einem Rohrsystem, das senkrecht oder waagrecht oder in beliebigem Winkeln dazwischen angeordnet sein kann, wobei das Rohrsystem sehr lang sein kann, vorzugsweise bis zu mehrere hundert Kilometer. Das Kultivierungsmedium wird dabei durch das Rohrsystem gefördert, vorzugsweise mittels von Pumpen oder des Airlift-Prinzips. Der Säulenbioreaktor besteht aus einem geschlossenen, zylindrischen Gefäß, welches mit dem Kultivierungsmedium befüllt ist. Bei Bioreaktoren dieses Typs wird Kohlendioxid eingeleitet, wobei die aufsteigende Blasensäule für die Durchmischung des Kultivierungsmediums sorgt. Bei Schlauchreaktoren handelt es sich um ein Reaktorsystem, welches aus einem einzigen Schlauch beliebiger Länge oder aus einer Vielzahl von Schläuchen beliebiger Länge, vorzugsweise von bis zu mehreren Metern langen Schläuchen besteht.

Die Bioreaktoren werden vorzugsweise aus transparenten oder transluzenten, additionsvernetzten Siliconmaterialien gefertigt. Unter transparenten Siliconmaterialien werden solche verstanden, welche mindestens 80 % des Lichtes im Spektralbereich von 400 nm bis 1000 nm hindurchlassen. Unter transluzenten Siliconmaterialien werden solche verstanden, welche mindestens 50 % des Lichtes im Spektralbereich von 400 nm bis 1000 nm hindurchlassen. Unter Reaktorteilen werden die Reaktorwandungen einschließlich Reaktorboden und Reaktorabdeckung sowie strukturgebende Elemente im Kultivierungsmedium, wie z.B. Strömungsbrecher verstanden. Den Reaktorwandungen entsprechen bei Rohr-, Platten- und Schlauchreaktoren, die Rohre, Platten und Schläuche. Die Reaktorwandungen werden ganz oder teilweise aus Siliconen gefertigt. Bei Rohrreaktoren oder Plattenreaktoren werden die Rohre oder Platten aus additionsvernetzten Siliconen gefertigt. Bei Säulenreaktoren werden die zylindrischen Behältnisse aus additionsvernetzten Siliconen gefertigt.

Zur Herstellung von Bioreaktoren geeignete Silicone sind additionsvernetzende Silicone, wobei die Additionsvernetzung thermisch oder mittels Strahlung eingeleitet werden kann. Peroxidisch vernetzte Silicone haben den Nachteil, dass diese Silicone im vernetzten Zustand eine höhere Klebrigkeit als additionsvernetzte Silicone aufweisen.

Additionsvernetzende Siliconkautschuk-Systeme enthalten
a) Organosiliciumverbindungen, die Reste mit aliphatischen Kohlenstoff-Kohlenstoff-Mehrfachbindungen aufweisen,
b) gegebenenfalls Organosiliciumverbindungen mit Si-gebundenen Wasserstoffatomen oder anstelle von a) und b)
c) Organosiliciumverbindungen, die Reste mit aliphatischen Kohlenstoff-Kohlenstoff-Mehrfachbindungen und Si-gebundene Wasserstoffatome aufweisen,
d) die Anlagerung von Si-gebundenen Wasserstoff an aliphatische Mehrfachbindung fördernde Katalysatoren und
e) gegebenenfalls die Anlagerung von Si-gebundenem Wasserstoff an aliphatische Mehrfachbindung bei Raumtemperatur verzögernde Mittel.

Geeignete durch Additionsreaktion vernetzende Siliconkautschuke sind bei Temperaturerhöhung vernetzende Festsiliconkautschuke (HTV).

Additionsvernetzte HTV-Siliconkautschuke erhält man durch die Vernetzung von mehrfach mit ethylenisch ungesättigten Gruppen, vorzugsweise Vinylgruppen, substituierten Organopolysiloxanen mit mehrfach mit Si-H-Gruppen substituierten Organopolysiloxanen in Gegenwart von Platinkatalysatoren.

Vorzugsweise besteht eine der Komponenten der additionsvernetzenden HTV-2-Siliconkautschuke aus Dialkylpolysiloxanen der Struktur R3SiO[-SiR₂O]n-SiR₃ mit n ≥ 0, im allgemeinen mit 1 bis 4 C-Atomen im Alkylrest R, wobei die Alkylreste ganz oder teilweise durch Arylreste wie den Phenylrest ersetzt werden können und an einem oder an beiden Enden einer der endständigen Reste R durch eine polymerisierbare Gruppe wie die Vinylgruppe ersetzt ist. Es können aber auch Polymere mit seitenständigen bzw. mit seiten- und endständigen Vinylgruppen verwendet werden. Bevorzugt werden vinylendblockierte Polydimethylsiloxane der Struktur CH₂=CH₂-R₂SiO[-SiR₂O]ₙ-SiR₂-CH₂=CH₂ eingesetzt, sowie vinylendblockierte Polydimethylsiloxane der genannten Struktur, welche noch seitenständige Vinylgruppen tragen. Bei additionsvernetzenden HTV-Siliconkautschuken ist die zweite Komponente ein Copolymer aus Dialkylpolysiloxanen und Polyalkylhydrogensiloxanen mit der allgemeinen Formel R'₃SiO[-SiR₂O]ₙ-[SiHRO]ₘ-SiR'₃ mit m ≥ 0, n ≥ 0 und R in der oben angegebenen Bedeutung und der Maßgabe, dass mindestens zwei SiH-Gruppen enthalten sein müssen, wobei R' die Bedeutung von H oder R haben kann. Es gibt demnach Vernetzer mit seitenständigen und endständigen SiH-Gruppen, während Siloxane mit R'= H, die nur endständige SiH-Gruppen besitzen, auch noch zur Kettenverlängerung verwendet werden. Als Vernetzungskatalysatoren kommen Platinkatalysatoren zum Einsatz. HTV-Siliconkautschuke werden auch als Einkomponentensystem verarbeitet.

Geeignete Materialien sind auch vernetzte Siliconhybridmaterialien, wie in der WO 2006/058656 beschrieben, deren diesbezügliche Angaben Teil dieser Anmeldung sind (icorporated by reference).

Eine detaillierte Übersicht über Silicone, ihre Chemie, Formulierung und Anwendungseigenschaften findet sich beispielsweise in Winnacker/Küchler, "Chemische Technik: Prozesse und Produkte, Band 5: Organische Zwischenverbindungen, Polymere",Seite 1095-1213, Wiley-VCH Weinheim (2005).

Wesentlich für die Hemmung bzw. Verhinderung des Bewuchses mit Mikroorganismen ist die Morphologie der Oberfläche der Siliconformteile. Die Morphologie der Oberfläche wird über den Kontaktwinkel dieser Oberfläche zu Wasser bestimmt. Der erfindungsgemäße Kontaktwinkel wird durch die erfindungsgemäße Auswahl der Siliconmaterialien eingestellt. Auf weitere Maßnahmen zur Erhöhung des Kontaktwinkels, beispielweise Aufrauhung der Oberfläche (z.B. Nachahmung des sog. Lotuseffekts), wird vorzugsweise verzichtet. Eine solche Aufrauhung kann nämlich die Kultivierung der phototrophen Mikroorganismen stören. Bevorzugt sind Oberflächen mit Kontaktwinkeln zwischen 100° und 120°, besonders bevorzugt Oberflächen mit Kontaktwinkeln zwischen 100° und 115°, und ganz besonders bevorzugt Oberflächen mit Kontaktwinkeln zwischen 100° und 113°. Die Bestimmung des Kontaktwinkels der Oberfläche der Siliconformteile zu Wasser kann mittels dem Fachmann bekannter Methoden, beispielsweise gemäß DIN 55660-2 erfolgen, unter Einsatz von im Handel erhältlicher Messgeräte zur Bestimmung des Kontaktwinkels, beispielsweise den bei der Fa. Krüss erhältlichen Kontaktwinkel-Messsystemen.

Gegebenenfalls können die additionsvernetzten Silicone übliche Additive zur Haftvermittlung oder übliche Füllstoffe oder Fasermaterialien zur Verbesserung der Mechanik enthalten. Diese Additive werden vorzugsweise maximal in solchen Mengen eingesetzt, dass das Siliconformteil transparent bzw. transluzent bleibt. Es können auch lichtleitende Additive und lichtwellenverschiebende Additive zugegeben werden.

Die mit dem Kultivierungsmedium in Kontakt kommenden Reaktorteile, insbesondere die Reaktorwandungen, werden zumindest teilweise, vorzugsweise vollständig, aus den genannten additionsvernetzten Siliconen gefertigt. Die Fertigung kann mit den etablierten Technologien der Kunststoffverarbeitung erfolgen, die zur Herstellung von Formkörpern wie Platten, Schläuchen, Rohren oder Behältern beliebiger Formgestalt eingesetzt werden. Beispielsweise mittels Extrusion zur Herstellung von Platten, Rohren, Schläuchen, oder Spritzgießen.

Es können auch Laminate hergestellt werden, welche aus einem Verbund aus additionsvernetztem Siliconformteil und aus Glas- oder Kunststoff-Formteil bestehen, das heißt ein Laminat mit Materialien, welche bisher zur Herstellung von Bioreaktoren eingesetzt worden sind. Beispiele für herkömmliche Materialien für Bioreaktoren sind Glas oder Kunststoffe wie Polymethylmethacrylat (Plexiglas), Polyester wie PET, Polycarbonat, Polyamid, Polystyrol, Polyethylen, Polypropylen, Polyvinylchlorid. Mit derartigen Laminaten lassen sich Bioreaktoren fertigen, deren Innenseite, das heißt dem Kultivierungsmedium zugewandte Seite, aus additionsvernetztem Silicon besteht.

Die Bioreaktoren sind mit Reaktoreinbauten ausgestattet. Beispielsweise, zur Befüllung und Nährstoffversorgung, mit Zuleitungen und zur Produktabtrennung und Entleerung mit Ableitungen (z.B. für Salz- und Feedlösungen). Zur Kühlung und Beheizung können die Bioreaktoren gegebenenfalls mit Heiz-/Kühlvorrichtungen wie Wärmeaustauschern ausgestattet werden. Des weiteren können die Bioreaktoren noch Rühreinrichtungen und Pumpen zur Durchmischung enthalten. Vielfach sind die Bioreaktoren noch mit Einrichtungen zur künstlichen Beleuchtung ausgestattet. Weitere Beispiele für Reaktoreinrichtungen sind Mess- und Steuergeräte für die Betriebsüberwachung (z.B. Analyse von pH, O₂, CO_{z}, Ionenleitstärke, Lichtstärke). In einer bevorzugten Ausführungsform werden die Reaktoreinbauten ganz oder teilweise noch mit Silicon beschichtet.

Die aus additionsvernetzendem Siliconformteilen, mit Oberflächen mit einem Kontaktwinkel zu Wasser mit Werten von mindestens 100°, hergestellten Photobioreaktoren minimieren die Ablagerung der sich bildenden phototrophen Organismen, so dass die Strömungsbedingungen des Kultivierungsmediums konstant bleiben, und die für das Wachstum ideale Lichteintragsmenge auf das Wachstumsoptimum eingestellt bleibt. Die Oberflächenbeschaffenheit der erfindungsgemäßen Siliconformteile mit einem Kontaktwinkel zu Wasser von mindestens 100°, reduziert zum einen die Anlagerung von Wasser auf der Siliconoberfläche, zum anderen werden auch in Wasser gelöste Stoffe, die z.B. durch Stresssituationen während der Algenkultivierung entstehen, von der Oberfläche ferngehalten.

Außerdem wird eine Minimierung des Reinigungsaufwands zwischen einzelnen Kultivierungszyklen sowie bei einem Wechsel der zu kultivierenden phototrophen Organismen erhalten. Gegebenenfalls an den Flächen anhaftende Organismen können zwischen den Kultivierungszyklen durch Absprühen mit Reinigungsmittel, z.B. Wasser, Ethanol, H₂O₂ ohne mechanischen Zusatzaufwand entfernt werden. Dies führt zu wesentlichen ökonomischen Vorteilen aufgrund kürzerer Stillstandzeiten und geringerem Reinigungsaufwand. Ein weiterer Vorteil ist die hohe UV-Stabilität von additionsvernetzten Siliconen mit der erfindungsgemäßen Oberflächenbeschaffenheit, welche insbesondere im Outdoor-Bereich die Standzeiten von Bioreaktoren aus additionsvernetzten Siliconmaterialien mit der erfindungsgemäßen Oberflächenbeschaffenheit gegenüber aus herkömmlichen Kunststoffen gefertigten Bioreaktoren deutlich erhöht.

## Patentansprüche

1. Bioreaktor zur Kultivierung von phototrophen Organismen in einem wässrigen Kultivierungsmedium, dessen mit dem Kultivierungsmedium in Kontakt kommende Reaktorwandungen, ganz oder teilweise, aus Siliconmaterialien gefertigt sind, **dadurch gekennzeichnet, dass** die Siliconmaterialien aus additionsvernetzten Siliconen gefertigt sind, und die Oberfläche der Siliconmaterialien einen Kontaktwinkel zu Wasser von mindestens 100° aufweist.

2. Bioreaktor nach Anspruch 1, **dadurch gekennzeichnet, dass** der Bioreaktor ein geschlossener Reaktor ist.

3. Bioreaktor nach Anspruch 1 oder 2, **dadurch gekennzeichnet, dass** der Bioreaktor ein Plattenbioreaktor, Rohrbioreaktor, (Blasen)Säulenbioreaktor oder Schlauchbioreaktor ist.

4. Bioreaktor nach Anspruch 1 bis 3, **dadurch gekennzeichnet, dass** die Reaktorwandungen aus einem oder mehreren Siliconen aus der Gruppe enthaltend additionsvernetzte Silicone (Siliconkautschuke) sowie additionsvernetzte Siliconhybridpolymere gefertigt sind.

5. Bioreaktor nach Anspruch 1 bis 4, **dadurch gekennzeichnet, dass** die Reaktorwandungen aus einem oder mehreren Siliconen aus der Gruppe enthaltend additionsvernetzte HTV-Siliconkautschuke sowie additionsvernetzte Siliconhybridpolymere gefertigt sind.

6. Verfahren zur Herstellung von Bioreaktoren, wobei die mit dem Kultivierungsmedium in Kontakt kommenden Reaktorwandungen, ganz oder teilweise, aus additionsvernetzten Siliconmaterialien gefertigt werden, deren Oberflächen einen Kontaktwinkel zu Wasser von mindestens 100° aufweisen.

7. Verfahren zur Herstellung von Bioreaktoren, wobei die Reaktorwandungen aus additionsvernetzten Siliconen mittels Extrusion, Spritzgießen oder Laminierung gefertigt werden.

8. Verwendung von additionsvernetzten Siliconformteilen, deren Oberfläche einen Kontaktwinkel zu Wasser von mindestens 100° aufweist, zur Herstellung von Reaktorwandungen für Bioreaktoren.

## Claims

1. Bioreactor for the cultivation of phototrophic organisms in an aqueous culture medium, in which reactor walls coming into contact with the culture medium are made completely or partially from silicone materials, **characterized in that** the silicone materials are made from addition-crosslinked silicones, and the surface of the silicone materials has a contact angle with water of at least 100°.

2. Bioreactor according to Claim 1, **characterized in that** the bioreactor is a closed reactor.

3. Bioreactor according to Claim 1 or 2, **characterized in that** the bioreactor is a plate-type bioreactor, tubular bioreactor, (bubble) column bioreactor or hose-type bioreactor.

4. Bioreactor according to Claims 1 to 3, **characterized in that** the reactor walls are made of one or more silicones from the group containing addition-crosslinked silicones (silicone rubbers) and addition-crosslinked silicone hybrid polymers.

5. Bioreactor according to Claims 1 to 4, **characterized in that** the reactor walls are made of one or more silicones from the group containing addition-crosslinked HTV silicone rubbers and addition-crosslinked silicone hybrid polymers.

6. Method of production of bioreactors, wherein the reactor walls coming into contact with the culture medium are made completely or partially from addition-crosslinked silicone materials, the surfaces of which have a contact angle with water of at least 100°.

7. Method of production of bioreactors, wherein the reactor walls are made from addition-crosslinked silicones by extrusion, injection molding or lamination.

8. Use of addition-crosslinked silicone moldings, the surface of which has a contact angle with water of at least 100°, for making reactor walls for bioreactors.

## Revendications

1. Bioréacteur pour la culture d'organismes phototrophes dans un milieu de culture aqueux, dont les parois du réacteur entrant en contact avec le milieu de culture sont constituées, en partie ou en totalité, de matériaux silicone, **caractérisé en ce que** les matériaux silicone sont constitués de silicones réticulés par addition, et la surface des matériaux silicone présente un angle de contact avec l'eau d'au moins 100°.

2. Bioréacteur selon la revendication 1, **caractérisé en ce que** le bioréacteur est un réacteur fermé.

3. Bioréacteur selon la revendication 1 ou 2, **caractérisé en ce que** le bioréacteur est un bioréacteur à plaques, un bioréacteur tubulaire, un bioréacteur de type colonne (à barbotage) ou un bioréacteur à poche.

4. Bioréacteur selon l'une quelconque des revendications 1 à 3, **caractérisé en ce que** les parois du réacteur sont constituées d'un ou de plusieurs silicones choisis dans le groupe contenant des silicones réticulés par addition (caoutchoucs silicone) ainsi que des polymères hybrides de silicones réticulés par addition.

5. Bioréacteur selon l'une quelconque des revendications 1 à 4, **caractérisé en ce que** les parois du réacteur sont constituées d'un ou de plusieurs silicones choisis dans le groupe contenant des caoutchoucs silicone-HTV réticulés par addition ainsi que des polymères hybrides de silicones réticulés par addition.

6. Procédé pour la fabrication de bioréacteurs, dans lequel les parois de réacteurs qui entrent en contact avec le milieu de culture sont constituées, en partie ou en totalité, de matériaux silicone réticulés par addition, dont les surfaces présentent un angle de contact avec l'eau d'au moins 100°.

7. Procédé pour la fabrication de bioréacteurs, dans lequel les parois des réacteurs sont fabriquées par extrusion, moulage par injection ou stratification à partir de silicones réticulés par addition.

8. Utilisation de pièces moulées à base de silicone réticulé par addition, dont la surface présente un angle de contact avec l'eau d'au moins 100°, pour la fabrication de parois de réacteurs pour bioréacteurs.
